# DEMANDE DE BREVET EUROPEEN

(11) **EP 0 798 308 A1**
(43) Date de publication de la demande: **01.10.1997**
(21) Numéro de dépôt: 97400569.6
(22) Date de dépôt: 14.03.1997
(51) Int. Cl.: C07H 11/04, C07H 13/06, C11D 1/66, C09K 3/16, D06M 13/10, A61K 7/48, A61K 7/50, A61K 7/08

(54) **Esters phosphoriques d'alkyl ou d'acyl dianhydro-1,4:3,6-d-glucitol, procédé de préparation et utilisations**

(30) Priorité: 27.03.1996 FR 9603813
(71) Demandeur: CECA S.A., 92800 Puteaux (FR)
(72) Inventeur: Petit, Serge, 74540 Cusy (FR); Fouquay, Stéphane, 76130 Mont-Saint-Aignan (FR); Bernard, Daniel, 92400 Courbevoie (FR)
(74) Mandataire: Haicour, Philippe

(57) **Abrégé**

L'invention a pour objet des composés de formule : 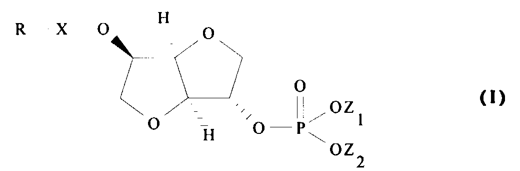 ou

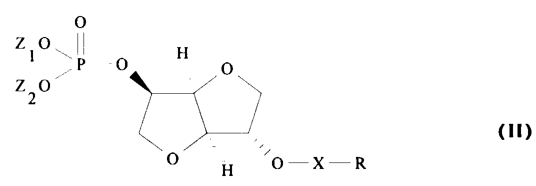

Elle concerne également un procédé de préparation desdits composés qui consiste à :
a) faire réagir du dianhydro-1,4:3,6-D-glucitol avec un composé de formule RCOOR' ou RCH₂Y en présence d'un catalyseur pour former les composés de formule : 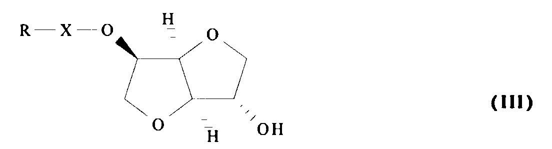 et 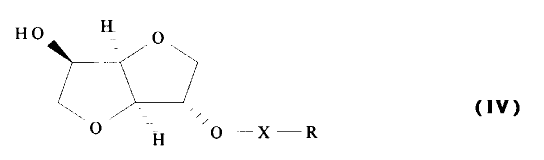 dans lesquelles :
   Y représente un halogène, un radical alkyle ou arylsulfonique,
   R' représente un atome d'hydrogène ou un radical alkyle ayant de 1 à 6 atomes de carbone
   R représente un radical alkyle linéaire ou ramifié, saturé ou insaturé, renfermant de 5 à 21 atomes de carbone
   X représente CO ou CH₂.
b) faire réagir le produit de l'étape a) avec de l'oxychlorure de phosphore, une base et un solvant anhydre, pour former les composés de formule (I) et/ou (Il),
   et, éventuellement les séparer.

Les composés, objet de l'invention, sont utilisables dans le domaine des tensio-actifs, notamment pour la préparation de compositions capillaires ou cosmétiques.

## Description

L'invention a pour objet de nouveaux esters phosphoriques du dianhydro-1,4:3,6-D-glucitol (ou isosorbide), leur procédé de préparation et leurs applications. Plus précisément, elle concerne les esters phosphoriques d'alkyl ou d'acyl isosorbide ayant des propriétés tensio-actives, leur procédé de préparation et leur utilisation, notamment pour préparer des compositions détergentes ou cosmétiques.

On sait que les greffages de substituants notamment alkyles ou acyles sur des glucides conduit à des composés présentant des propriétés tensio-actives, souvent spécifiques, et qui, en outre, sont biodégradables.

Les glucides de la série du dianhydro-1,4:3,6-D-glucitol, en particulier, ont fait l'objet de nombreuses études.

Ainsi, on a proposé de préparer des alkyloyl-dianhydro-1,4:3,6-D-glucitol par estérification chimique sélective en position 2 ou 5 [voir par exemple CEKOVIC Z. et TOKIEC Z., Synthesis, pp. 610-612 (1989); LE LEM G. et al., Bull. Soc. Chim. Fr., n°3, pp. 567-570 (1988); ABENHAÏM D. et al., Carbohyd. Res., 261, pp. 255-266 (1994)]. Ces composés sont utilisés notamment comme intermédiaires dans la synthèse de vasodilatateurs (MONTS-2 et MONTS-5).

MUKESH D. et al. [Biotech. Lett. vol 15 n°2, pp 1243-1246 (1993)] décrivent un mélange de monooléates de dianhydro-1,4:3,6-D-glucitol obtenu par estérification catalytique au moyen d'une lipase immobilisée.

ZARIF L. et al. [J.Fluor. Chem. vol 44, pp. 73-85 (1989)] proposent de préparer des mono-esters perfluoroalkylés du dianhydro-1,4:3,6-D-glucitol utilisables dans le domaine biomédical (substituts du sang).

JAZINSKI W. et ROPUSZYNSKI S. [CA 79 : 146 767 h; CA 80: 61 387e; CA 80: 146 h] proposent un mélange comprenant les dérivés 2-(triNa pyrophosphate), 2-(diNa pyrophosphate) et 2-phosphate du 5-acyl-dianhydro-1,4:3,6-D-gluci-tol, dans lequel le radical acyle représente un radical oléyle, stéaryle ou lauryle.

Enfin, SAHEKI et al. [JAOCS, vol. 63 n°7, pp. 927-930 (1986)] proposent des 2-alkyloyl-dianhydro-1,4:3,6-D-glucitol-5-sulfates dont le radical alkyloyl renferme de 8 à 16 atomes de carbone. De tels composés peuvent être utilisés comme agents tensio-actifs.

Dans le domaine des tensio-actifs, le besoin est grand de rechercher des composés dont la résistance à l'hydrolyse et la compatibilité avec la peau et les muqueuses sont améliorées.

La présente invention a donc pour objet de nouveaux esters phosphoriques de formule : 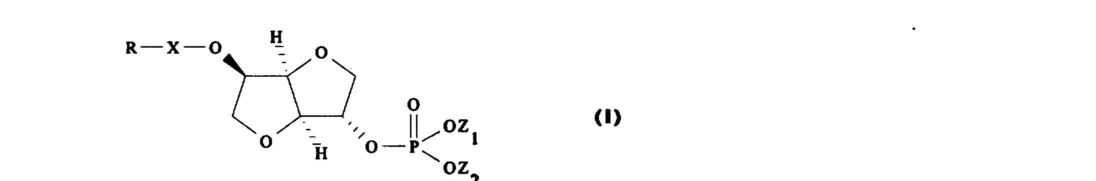 ou 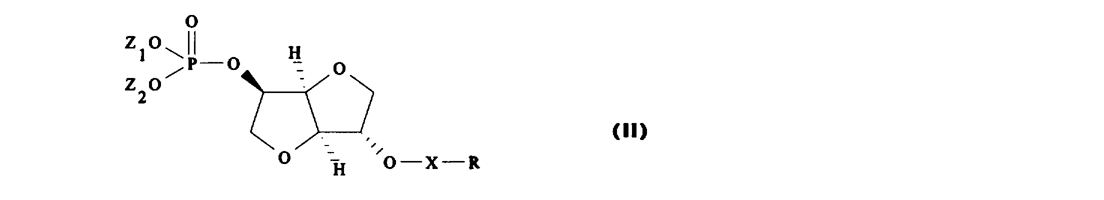 dans laquelle :
R représente un radical alkyle, linéaire ou ramifié, saturé ou insaturé, renfermant de 5 à 21 atomes de carbone,
X représente CO ou CH₂,
Z₁ et Z₂, identiques ou différents, représentent un atome d'hydrogène, un métal alcalin ou alcalino-terreux ou un ammonium quaternaire de formule : 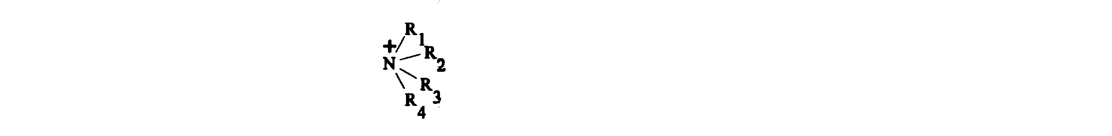 dans laquelle R₁, R₂, R₃ et R₄, identiques ou différents, représentent un atome d'hydrogène, un radical alkyle ou hydroxyalkyle renfermant de 1 à 6 atomes de carbone ou un résidu d'amino-acide basique.

L'invention a également pour objet un procédé de préparation des composés précités de formule (I) ou (Il) qui consiste:
a) à faire réagir du dianhydro-1,4:3,6-D-glucitol avec un composé de formule RCOOR' ou RCH₂Y en présence d'un catalyseur pour former les composés de formule : 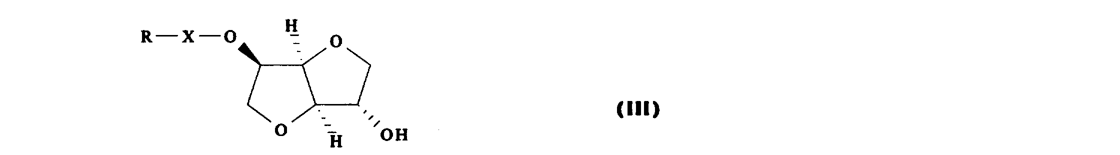 et 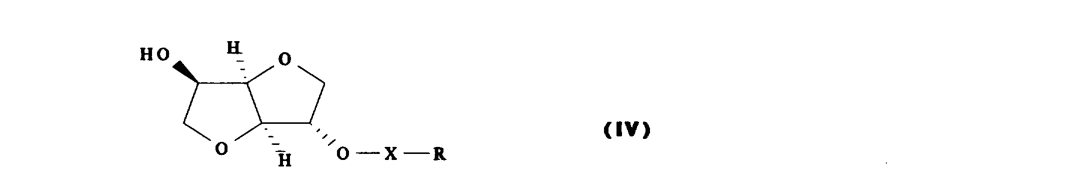 dans lesquelles :
   Y représente un halogène, un radical alkyle ou arylsulfonique,
   R' représente un atome d'hydrogène ou un radical alkyle ayant de 1 à 6 atomes de carbone,
   R et X ont la signification donnée précédemment, éventuellement séparer lesdits composés (III) et (IV).
**b)** et faire réagir le produit de l'étape a) avec de l'oxychlorure de phosphore, une base et un solvant anhydre.

Un autre objet de l'invention concerne l'application des composés précités de formule (I) et (Il) en tant qu'agents tensio-actifs.

Un autre objet de la présente invention concerne encore les compositions notamment détergentes ou cosmétiques comprenant les composés précités.

Le procédé de préparation des composés selon l'invention est décrit de manière détaillée dans ce qui suit :

### ETAPE A: PREPARATION DES COMPOSES DE FORMULE (III) ET (IV)

### 1. Préparation des esters (X = CO)

Les esters de formule (III) et (IV) sont obtenus en faisant réagir le dianhydro1,4:3,6-D-glucitol avec un acide gras ou un ester d'acide gras de formule RCOOR' en présence d'un catalyseur.

L'acide gras est choisi parmi les acides gras à chaîne linéaire ou ramifiée, saturée ou insaturée, renfermant de 6 à 22 atomes de carbone, de préférence 7 à 18 atomes de carbone.

L'ester d'acide gras est choisi parmi les esters des acides gras précités dont le groupement ester comprend de 1 à 6 atomes de carbone, et de préférence 1 à 2.

Le catalyseur varie selon la nature du composé de formule RCOOR'.

Lorsqu'on met en oeuvre un acide gras, le catalyseur est constitué par un acide. A titre d'illustration, on peut citer l'acide chlorhydrique, l'acide sulfurique, les acides alkyl sulfuriques, par exemple l'acide décyl ou lauryl sulfurique, les acides sulfoniques, par exemple l'acide benzène sulfonique, paratoluène sulfonique ou camphrosulfonique, les acides alkyl sulfoniques, par exemple l'acide méthan***o(e ?)*** sulfonique, décyl sulfonique, lauryl sulfonique, sulfosuccinique ou un sulfosuccinate d'alkyle tel que le sulfosuccinate de décyle ou de lauryle, les acides perhalohydriques, par exemple l'acide perchlorique, l'acide hypophosphoreux ou les mélanges de ces acides. De préférence, on utilise l'acide sulfurique, un acide alkyl sulfurique, l'acide méthane sulfonique, l'acide succinique ou un sulfosuccinate d'alkyle, l'acide hypophosphoreux ou les mélanges de ces acides.

Lorsqu'on met en oeuvre un ester d'acide gras, le catalyseur est une base. A titre d'illustration d'une telle base, on peut citer les alcoolates métalliques tels que les méthylates ou les éthylates d'un métal alcalin tel que le sodium ou le potassium, les carbonates, les hydrogénocarbonates par exemple d'un métal alcalin tel que le sodium ou le potassium, les composés de formule M(OH)ₓ dans laquelle M est un métal alcalin ou alcalino-terreux et x est la valence du métal, les alumines basiques, les hydroxydes d'ammonium quaternaire de formule : 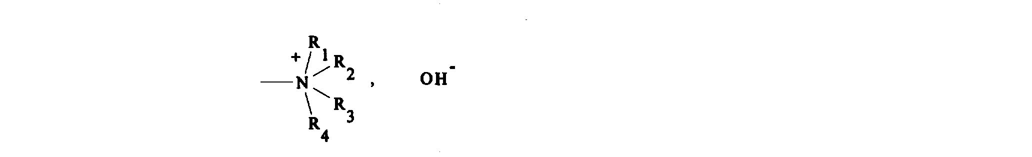 dans laquelle R₁, R₂, R₃ et R₄, identiques ou différents, représentent un radical alkyle ou alkylaryle renfermant de 1 à 18 atomes de carbone, ou les mélanges de ces bases.

De manière avantageuse, on peut ajouter à la base précitée un catalyseur de transfert de phase choisi parmi les catalyseurs connus tels que, par exemple, les halogénures de tétrabutylammonium (notamment le bromure) ou de méthyltrioctyl ammonium (notamment le chlorure).

Le catalyseur précité est généralement mis en oeuvre à raison de 0,1 à 20 %, et de préférence 1 à 5 %, en poids du dianhydro-1,4:3,6-D-glucitol de départ.

On peut réaliser la préparation des esters précités en présence d'un solvant et ce, que ce soit à partir de l'acide gras ou de l'ester de cet acide. A titre d'exemple, on peut citer les alcanes, les étheroxydes tels que le tétrahydrofuranne, le dioxanne ou l'éther diméthylique du diéthylène glycol, les hydrocarbures halogénés tels que le dichlorométhane, le chloroforme ou le dichloroéthane, les solvants du type amide tels que le N-méthyl formamide, le N,N-diméthyl formamide, le N,N-diméthyl acétamide ou la N-méthyl 2-pyrrolidone, les nitriles tels que l'acétonitrile, les sulfoxydes tels que le diméthyl sulfoxyde, les solvants aromatiques tels que le toluène ou le xylène, et les mélanges de ces solvants.

Dans la préparation des esters selon l'invention, on utilise en général de 0,1 à 20 équivalents en mole, et de préférence 0,5 à 10 équivalents, de dianhydro1,4:3,6-D-glucitol, de 0,5.10⁻³ à 2 équivalents en mole de catalyseur acide ou basique, et de préférence 1.10⁻³ à 1.10⁻¹ équivalent (catalyseur acide) ou 1.10⁻² à 1 équivalent (catalyseur basique), et de 0 à 20 équivalents en poids, et de préférence 0 à 10 équivalents de solvant, calculé sur la base d'un équivalent en poids d'acide gras ou d'esters dudit acide de départ.

La réaction est généralement mise en oeuvre à une température comprise entre 25 et 200°C, et de préférence 70 à 180°C, pendant une durée pouvant varier de 1 minute à 48 heures, et de préférence 2 minutes à 24 heures et sous une pression comprise entre 0,013 et 101,325 kPa.

De manière avantageuse, notamment pour diminuer la durée, la réaction est effectuée sous une irradiation micro-onde.

A l'issue de la réaction, le milieu réactionnel est généralement filtré afin d'éliminer le catalyseur. Le filtrat ainsi obtenu contient les esters de formule (III) et (IV), les composés peuvent éventuellement être séparés, par exemple par chromatographie sur une colonne de silice ou par précipitation et/ou recristallisation dans un solvant choisi parmi les solvants précités et les solvants de la famille des esters tels que l'acétate d'éthyle, de propyle ou de butyle.

### 2. Préparation des éthers (X = CH₂)

Les éthers de formule (III) et (IV) sont préparés en faisant réagir le dianhydro-1,4:3,6-D-glucitol avec un composé de formule RCH₂Y en présence d'un catalyseur.

Le composé de formule RCH₂Y dans laquelle R et Y sont définis tels que précédemment, est de préférence, choisi parmi les composés contenant un atome de chlore ou un radical sulfonyle tel que le méthanesulfonyle et le paratoluènesulfonyle.

Le catalyseur est choisi parmi les bases définies précédemment, les métaux alcalins et les hydrures de ces métaux. De préférence, on utilise le sodium, le potassium et l'hydrure de sodium ou de lithium.

De manière avantageuse, on peut ajouter au catalyseur précité, un catalyseur de transfert de phase choisi parmi les sels d'ammonium quaternaire tels que les halogénures de tétraalkyl ou tétraarylalkyl ammonium, par exemple le chlorure ou le bromure de tétrabutylammonium, les hydrogénosulfates d'ammonium, par exemple l'hydrogénosulfate de méthyltrioctyl, benzyltriméthyl ou benzyltriéthyl ammonium.

Ce catalyseur peut, en outre, contenir des sels tels que les halogénures ou les perchlorates d'un métal alcalin, par exemple de rubidium ou de césium.

On peut, en outre, effectuer la réaction en présence d'un des solvants précités, d'eau et des mélanges miscibles ou non miscibles de ces composés.

Dans la préparation des éthers selon l'invention, on utilise en général de 1 à 20 équivalents en mole, et de préférence 1 à 5 équivalents, de dianhydro-1,4:3,6-Dglucitol, de 1 à 20 équivalents en mole, et de préférence 1 à 5 équivalents, de catalyseur et de 0 à 20 équivalents en poids, et de préférence 0 à 10 équivalents de solvant, calculé sur la base d'un équivalent en poids du composé de formule RCH₂Y.

La réaction est généralement mise en oeuvre à une température comprise entre 0 et 200°C, et de préférence 25 et 160°C, pendant une durée pouvant varier de 1 minute à 48 heures, et de préférence 2 minutes à 24 heures.

De manière avantageuse, notamment pour diminuer la durée, la réaction est effectuée sous une irradiation micro-onde.

A l'issue de la réaction, on récupère dans le milieu réactionnel les éthers de formule (III) et (IV). Ces éthers peuvent éventuellement être séparés par exemple par chromatographie sur une colonne de silice.

### ETAPE B : PREPARATION DES COMPOSES DE FORMULE (I) ET (II)

On fait réagir le produit de réaction de l'étape a) avec de l'oxychlorure de phosphore, une base et un solvant anhydre.

Le produit de réaction de l'étape a) est généralement constitué par le composé de formule (III) ou (IV) sous la forme soit d'un ester (X=CO) soit d'un éther (X=CH₂).

Toutefois, on peut utiliser ces composés sous la forme d'un mélange d'esters ou d'éthers ou d'esters et d'éther.

L'oxychlorure de phosphore est, en général, préalablement distillé.

La base est choisie parmi la pyridine, la N,N-diméthyl ou diéthylamino-4 pyridine et les amines tertiaires telles que la N-méthyl ou N-éthylmorpholine.

Le solvant anhydre est généralement choisi parmi les solvants cités précédemment. De préférence, on utilise les solvants chlorés tels que le chloroforme, le dichlorométhane ou le dichloroéthane et les étheroxydes tels que le tétrahydrofuranne, le dioxanne, l'éther diméthylique de l'éthylène ou du diéthylène glycol.

Dans la mise en oeuvre de l'étape b), du procédé selon l'invention, on utilise en général de 1 à 5 équivalents en mole, et de préférence 1 à 2 équivalents, d'oxychlorure de phosphore, de 1 à 5 équivalents en mole, et de préférence 1 à 5 équivalents, de la base et de 1 à 50 équivalents en poids, et de préférence 1 à 20 équivalents du solvant calculé sur la base d'un équivalent en poids du composé de formule (III) et/ou (IV) de départ.

La réaction est généralement réalisée à une température comprise entre -20 et +60°C, et de préférence -10 et +40°C, et pendant une durée variant de 1 heure à 7 jours, et de préférence 1 heure à 48 heures.

De manière avantageuse, la réaction est effectuée sous une atmosphère inerte et anhydre, par exemple sous un bullage d'argon et d'azote.

A l'issue de la réaction, le chlorure correspondant à la base utilisée est éliminé du milieu réactionnel par filtration. Le filtrat, auquel on ajoute éventuellement un solvant tel que défini précédemment, et de préférence un alcane (par exemple l'hexane ou l'heptane), est évaporé. Le résidu d'évaporation, auquel on ajoute 1 à 20 équivalents en poids d'eau glacée, est maintenu à une température comprise entre 0 et 40°C, de préférence 10 et 30°C pendant 30 minutes à 5 heures, de préférence 1 à 2 heures, sous agitation intense.

Le composé I ou Il présent dans la phase aqueuse ainsi obtenue peut être récupéré de plusieurs manières, par exemple :
- par concentration de ladite phase, notamment sous vide et à une température voisine de 60°C,
- par extraction de ladite phase avec au moins un solvant organique ad hoc et concentration de la (des) phase(s) organique(s),
- par basification de ladite phase et addition d'au moins un solvant capable de provoquer la précipitation du composé précité, de dernier pouvant alors être avantageusement récupéré par filtration, par exemple sur un verre fritté.

Le composé de formule (I) ou (Il) peut éventuellement subir une étape de purification supplémentaire, par exemple par chromatographie d'exclusion sur une colonne de gel tel que cyano-éconosil prepCN (90 Å; 15-35 µm ; ALLTECH) ou par chromatographie par échange d'ions sur une résine cationique forte telle que AMBERLITE IR 120H⁺, ou par recristallisation.

Les esters phosphoriques de formule (I) et (Il) présentent des propriétés tensio-actives, notamment solubilisantes, émulsionnantes, moussantes, mouillantes et dispersantes. En outre, les composés selon l'invention ont la propriété de ne pas agresser la peau et les muqueuses, ce qui les rend particulièrement adaptés pour la préparation de compositions destinées à l'hygiène, telles que des shampooings et des compositions capillaires ou cosmétiques, par exemple des pommades, des crèmes, des laits de beauté,...

Du fait de leur pouvoir moussant et de leur capacité à adoucir la peau, les composés selon l'invention peuvent aussi être utilisés dans des compositions pour le bain (bains moussants) ou la douche (gel douche), ainsi que dans des savons notamment de type syndet ("Synthetic Detergent" en anglais).

Les composés selon l'invention peuvent également être utilisés en tant qu'agents antistatiques pour le traitement des textiles, agents mouillants et détergents à utilisations spéciales, agents de mouillage pour le traitement des textiles et du cuir, et émulsifiants dans les domaines tels que la recherche pétrolière, le traitement des métaux et la polymérisation en suspension.

Les exemples qui suivent permettent d'illustrer l'invention. Dans les exemples, on utilise les méthodes d'analyse suivantes :
- le Rf est mesuré par chromatographie sur couche mince de silice (épaisseur du film : 200 µm ; taille des particules: 5-10 µm). Le solvant de migration est un mélange acétate d'éthyle/hexane 50/50 (v/v). Les spots de migration sont révélés par pulvérisation d'acide sulfurique à 50 % en volume dans l'eau et chauffage à 120°C pendant 2 minutes.
- résonance magnétique nucléaire (RMN) :
   - 1H : réalisée à 250 MHz en présence de CDCl₃. Les déplacements chimiques sont exprimés en ppm et les constantes de couplage (J) en Hz.
   - ¹³C réalisée à 75 MHz en présence de CDCl₃ (exemples 1-4, 7-8) ou D₂O (exemple 5). Les déplacements chimiques sont exprimés en ppm et les constantes de couplage (J) en Hz.

### EXEMPLE 1

Préparation des composés de formules (III-a) et (IV-a): X=CO et R=C₁₁H₂₃.

On mélange 1,52g (10,4 mmoles) de dianhydro-1,4:3,6-D-glucitoî (ROQUETTE Frères), 4,46g (20,8 mmoles) de dodécanoate de méthyle (FINA CHEMICALS), 0,94 g (6,8 mmoles) de carbonate de potassium, 0,1 g (0,31 mmole) de bromure de tétrabutylammonium et 2 ml de diméthylformamide.

Le mélange obtenu est soumis à une irradiation micro-ondes de 20 watts pendant 15 minutes sous agitation.

Après refroidissement, on solubilise le gâteau obtenu dans 20 ml d'acétate d'éthyle et on concentre la solution sous vide dans un évaporateur rotatif (60°C ; 2,4 kPa puis 0,1 kPa).

On chromatographie la solution ainsi concentrée sur une colonne (⌀= 2,5 cm ; longueur = 15 cm) remplie de silice (200-300 Mesh ASTM) éluée avec de l'heptane puis d'un mélange heptane/acétate d'éthyle 95/5 (v/v).

On élue successivement 0,586g de 2-O-dodécanoyl-dianhydro-1,4:3,6-D-glucitol (IV-a) et 1,67 g de 5-O-dodécanoyl-dianhydro-1,4:3,6-D-glucitol (III-a), soit un rendement calculé sur la base du dianhydro-1,4:3,6-D-glucitol de départ égal à 17,1 % et 48,9 % respectivement.

### EXEMPLE 2

Préparation des composés de formules (III-a) et (IV-a): X= CO et R= C₁₁H₂₃.

Un mélange comprenant 150g (748,8 mmoles) d'acide dodécanoïque (réf.15, 378-8; ALDRICH), 547g (3743 mmoles) de dianhydro-1,4:3,6-D-glucitoî (ROQUETTE Frères) et 1,75 g d'un mélange équimolaire d'acide méthanesulfonique et d'acide hypophosphoreux est chauffé à 160°C pendant 8 heures sous atmosphère d'azote.

Le milieu réactionnel obtenu (678 g) est solubilisé dans un litre d'heptane et placé à 4°C pendant 24 heures. Il se forme un précipité qui est récupéré par filtration et partiellement solubilisé dans de l'acétate d'éthyle (4 x 200 ml). Les phases organiques sont rassemblées, lavées à l'eau (2 x 100 ml), séchées (sulfate de magnésium), filtrées (verre fritté n°3) et concentrées dans un évaporateur rotatif. Le résidu d'évaporation est recristallisé dans le pentane.

Le composé recristallisé (104 g) est constitué de 2-O-dodécanoyl-dianhydro-1,4:3,6-D-glucitol (IV-a), soit un rendement calculé sur la base de l'acide dodécanoïque de départ égal à 42 %. La phase organique (heptane) est concentrée dans un évaporateur rotatif et le résidu obtenu est précipité dans le pentane. Après filtration, la phase organique est lavée à l'eau, séchée (sulfate de magnésium), filtrée et concentrée dans un évaporateur rotatif. On récupère 53 g de 5-O-dodécanoyl-dianhydro-1,4:3,6-D-glucitol (III-a), soit un rendement calculé sur la base de l'acide dodé-canoïque de départ égal à 21,6 %.

Les caractéristiques des composés (III-a) et (IV-a) sont présentées ci-après.

### Point de fusion

- composé (IV-a) : 78°C (pentane)
- composé (III-a) : huile

### Rf en chromatographie sur couche mince

- composé (IV-a) : 0,5
- composé (III-a) : 0,4

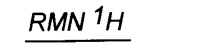

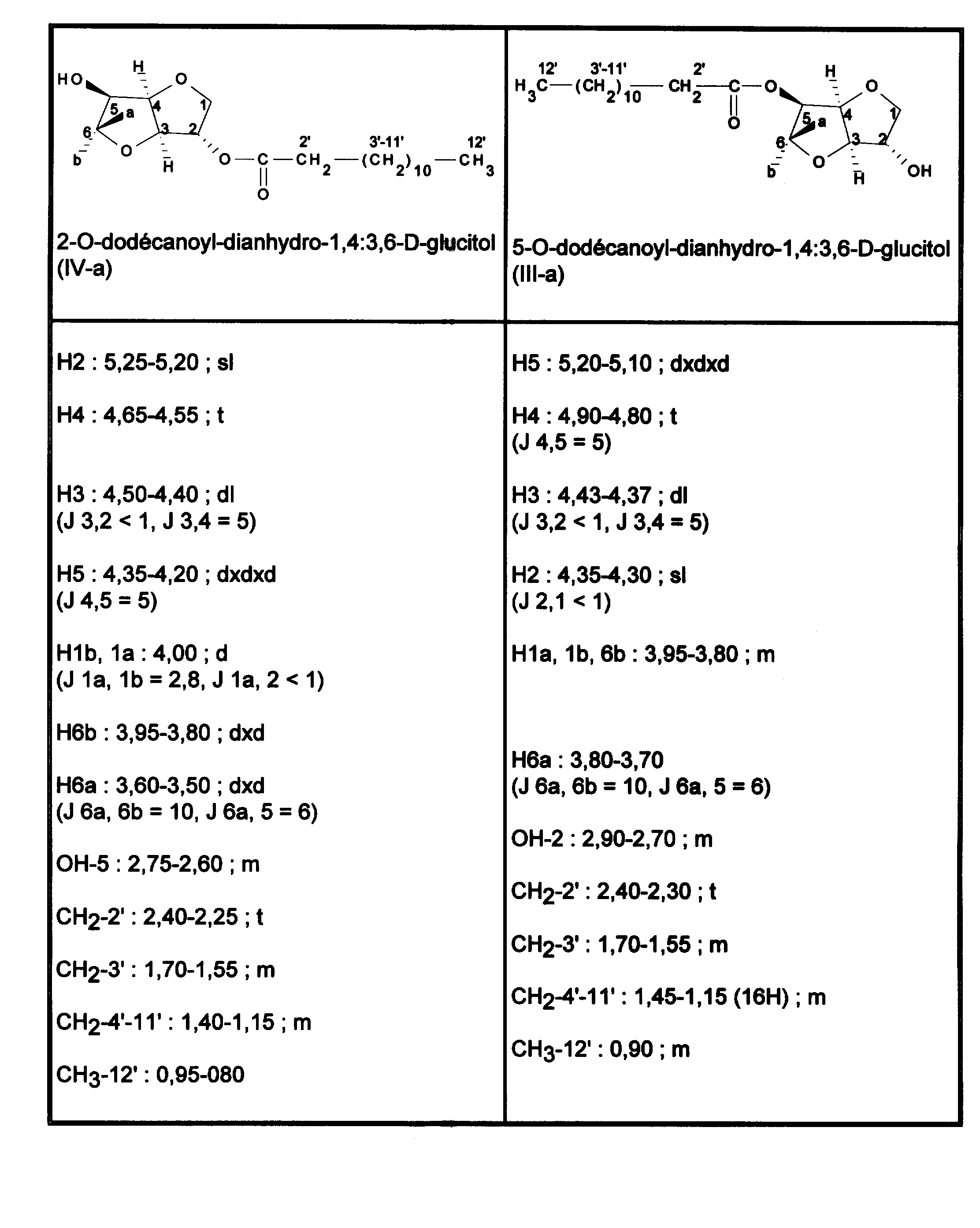

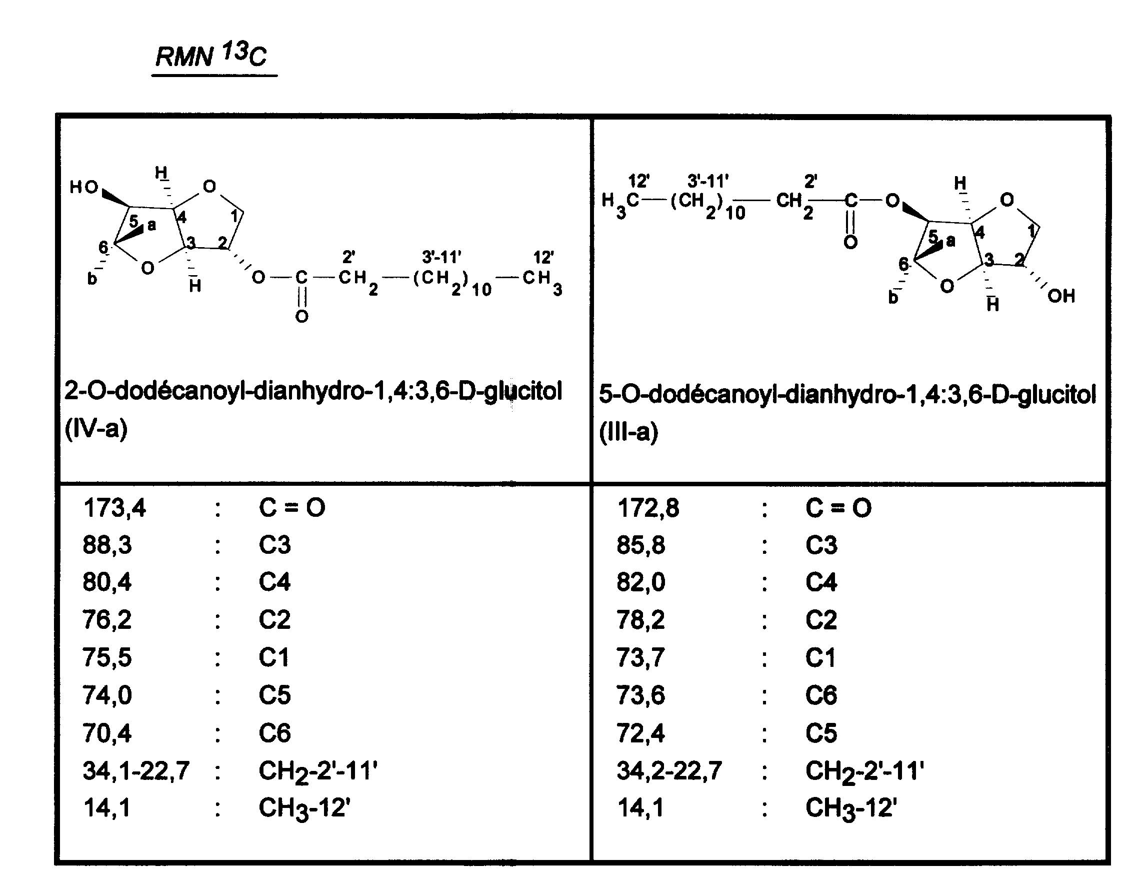

### EXEMPLE 3

Préparation des composés de formules (III-b) et (IV-b) : X=CH₂ et R=C₁₁H₂₃.

On solubilise 10 g (68 mmoles) de dianhydro-1,4:3,6-D-glucitol ***(ROQUETTE Frères)*** dans un mélange constitué de 35 ml de diméthylsulfoxyde et 10 ml d'eau permutée. On ajoute 5,8 g (89 mmoles) de potasse à la solution précitée et on place le mélange obtenu dans un bain d'huile à 90°C. On agite le mélange pendant 20 minutes et on ajoute, goutte à goutte, 13 g (52 mmoles) de bromododécane (B6, 551-1; ALDRICH).

Après 24 heures, on filtre le mélange réactionnel sur 10 ml de silice (230-400 Mesh ASTM). Le filtrat est concentré dans un évaporateur rotatif (60°C ; 2,4 kPa puis 0,01 kPa) et le résidu obtenu, repris par 100 ml d'un mélange eau/acétate d'éthyle 50/50 (v/v), est agité à 400 RPM pendant 5 minutes. La phase organique est récupérée, lavée à l'eau (2 x 20 ml), séchée (sulfate de magnésium) et concentrée dans un évaporateur rotatif (50°C ; 2,4 kPa).

Le résidu obtenu est adsorbé sur 5 ml de silice (230-400 Mesh ASTM) et chromatographié sur une colonne (⌀ = 5,6 cm ; longueur = 25 cm) remplie de la silice précitée munie d'un système de détection par réfractométrie différentielle. L'élution au moyen d'un gradient heptane/acétate d'éthyle (100/0 à 50/50 (v/v) en 30 minutes) permet de récupérer successivement 0,95 g de 2-O-dodécyl-dianhydro1,4:3,6-D-glucitol (IV-b) et 2,3 g de 5-O-dodécyl-dianhydro-1,4:3,6-D-glucitol (III-b) soit un rendement calculé sur la base du bromododécane de départ égal à 6,1 et 14,0 % respectivement.

### EXEMPLE 4

Préparation des composés de formules (III-b) et (IV-b): X=CH₂ et R=C₁₁H₂₃.

Dans un ballon de 250 ml muni d'un système d'agitation mécanique, d'un réfrigérant et d'une garde (CaCl₂), on introduit 43,9 g (0,3 mole) de dianhydro-1,4:3,6-D-glucitol (ROQUETTE Frères). On chauffe le ballon à 90°C et on ajoute, de manière fractionnée, 1g (0,12 mole) d'hydrure de lithium. Après une heure, on ajoute, goutte à goutte, 24,9 g (0,1 mole) de bromododécane (20,104-9; ALDRICH) on agite pendant 3 heures à 140°C et on refroidit à 60°C. Le mélange est transféré dans un ballon d'un litre et on ajoute précautionneusement 100 ml d'eau permutée et 200 ml d'acétate d'éthyle. On agite pendant 5 minutes et on récupère la phase organique qui est lavée à l'eau (2 x 50 ml), séchée (sulfate de magnésium) et concentrée dans un évaporateur rotatif. On obtient 21,4 g d'une huile brune que l'on chromatographie sur une colonne (⌀ = 5,8 cm ; longueur = 25 cm) remplie de silice (230-400 Mesh ASTM) munie d'un système de détection par réfractométrie différentielle. La colonne est éluée au moyen d'un mélange acétate d'éthyle/hexane 50/50 (v/v) à raison de 40 ml/min.

On récupère successivement 3 g de 2-O-dodécyl-dianhydro-1,4:3,6-D-gluci-tol (IV-b), 5g de 5-O-dodécyl-dianhydro-1,4:3,6-D-glucitol (III-b) et 1,6g d'un mélange des deux composés précités (IV-b et III-b) soit un rendement calculé sur la base du bromododécane de départ égal à 9,5, 16 et 5 % respectivement.

Les caractéristiques des composés (IV-b) et (III-b) sont présentés ci-après.

### Point de fusion

- composé (IV-b) : huile
- composé (III-b) : 55°C (pentane)

### Rf

- composé (IV-b) : 0,60 (acétate d'éthyle/heptane)
   0,45 (acétate d'éthyle/hexane)
- composé (III-b) : 0,40 (acétate d'éthyle/heptane)
   0,26 (acétate d'éthyle/hexane)

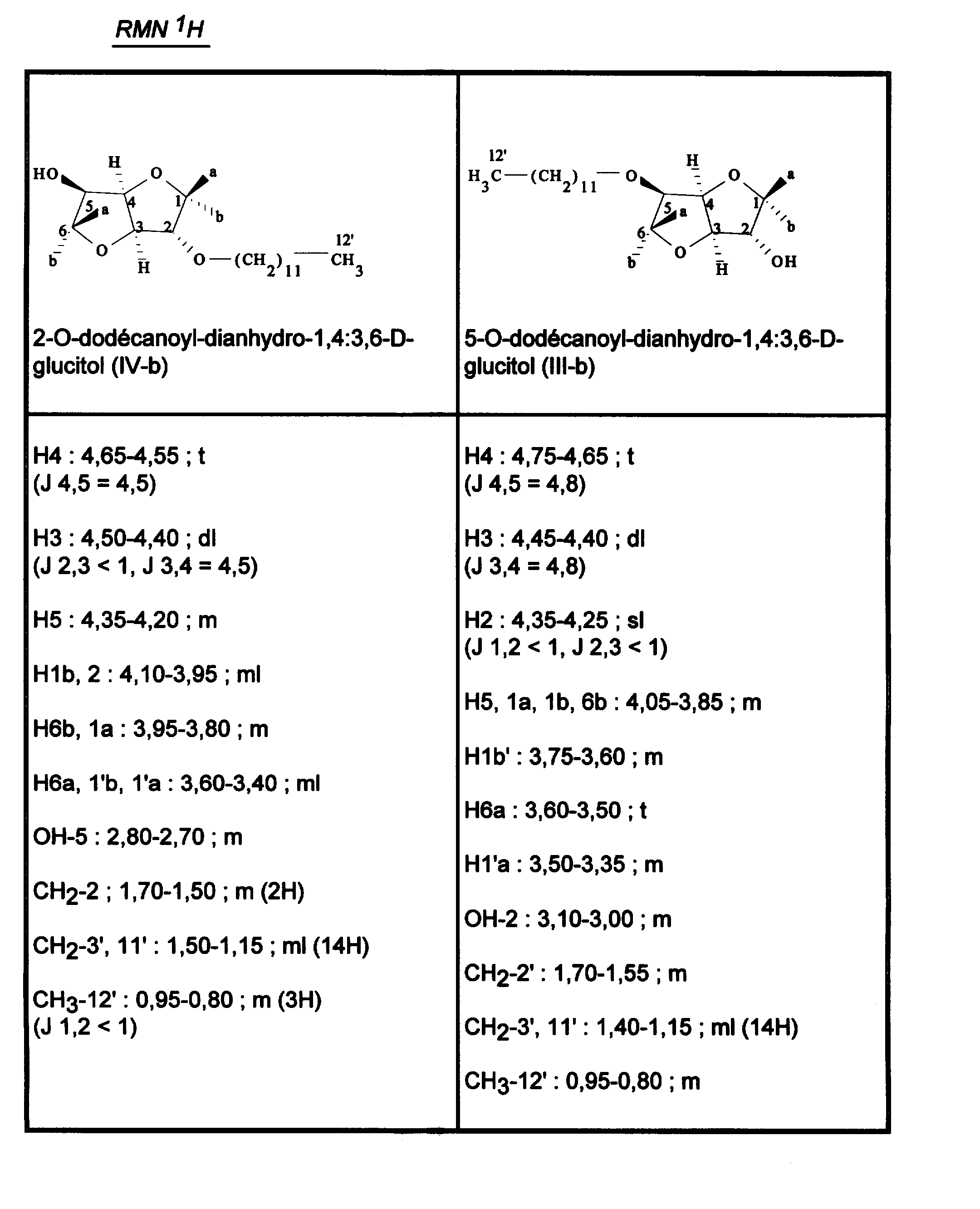

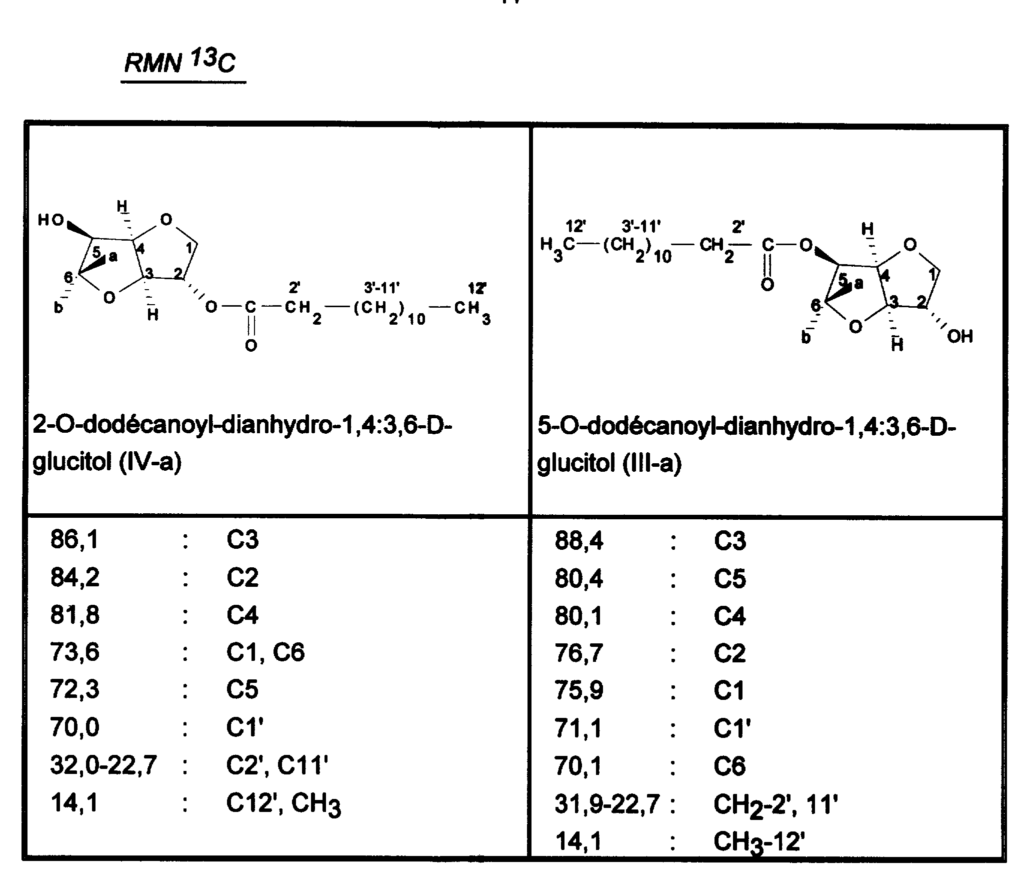

### EXEMPLE 5

Préparation du composé (Il-a) : X=CO ; R=C₁₁H₂₃ et Z₁=Z₂=H.

On solubilise 1g (3,04 mmoles) de 2-0-dodécanoyl-dianhydro-1 ,4:3,6-D-glucitol (composé IV-a) de l'exemple 1 dans 10 ml de dichlorométhane distillé et anhydre (lavage à l'eau, séchage avec CaCl₂, distillation sur P₂O₅). On place la solution à 0°C sous atmosphère d'argon et on ajoute 0,385 g (3,34 mmoles) de N-éthyl morpholine distillée et, goutte à goutte, 0,469 g (3,05 mmoles) d'oxychlorure de phosphore distillé. Le mélange obtenu devient jaune.

Ce mélange, maintenu à 20°C sous agitation pendant 4 jours, est concentré dans un évaporateur rotatif. Le résidu d'évaporation est mis en suspension dans 20 ml d'heptane à 0°C, agité pendant 10 minutes et filtré sur verre fritté (porosité 3).

Le filtrat ainsi récupéré, maintenu à 0°C, est hydrolysé à l'aide de 20 ml d'une solution tampon aqueuse (acide acétique/acétate de sodium ; pH = 4,75).

Après agitation pendant une heure à 20°C, on concentre le mélange dans un évaporateur rotatif (60°C ; 2,4 kPa).

Le résidu d'évaporation est purifié par chromatographie sur une colonne CYANOECONOSIL PREP-CN (ALLTECH; diamètre de pore: 90Å; taille des particules: 15-35 µm) éluée au moyen d'un gradient acétate d'éthyle/hexane (0/100 à 50/50 (v/v)) et acétate d'éthyle/éthanol (90/10 à 0/100 (v/v)).

Après concentration des phases organiques dans un évaporateur rotatif, on récupère une mousse pâteuse jaunâtre contenant 0,281 mg de 2-0-dodécanoyl-dianhydro-1,4:3,6-D-glucitol-5-phosphate (Il-a), soit un rendement calculé sur la base du composé (IV-a) de départ égal à 22 %.

Les caractéristiques du composé (Il-a) sont présentées ci-après.

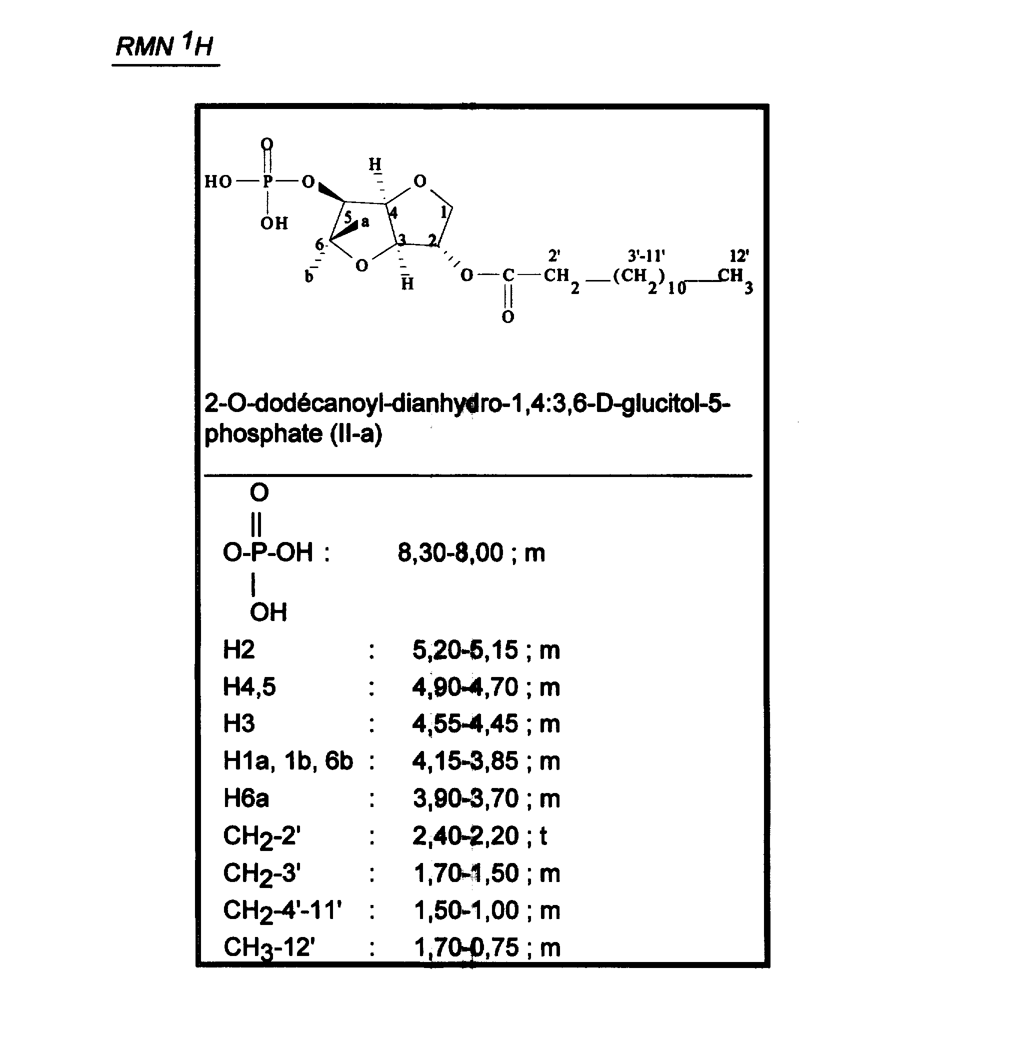

### RMN ³¹P : référence H₃PO₄

Pic phosphate à 0 ppm.

### RMN ¹³C : sel de potassium

- CO :: 184,57
- C₁-C₆ :: 87,07 ; 81,54 ; 76,07 ; 75,05 ; 74,08 ; 69,99 ;
- (CH₂)ₙ-2'-11':: 38,25 ; 31,94 ; 29,61; 29,39 ; 29,31 ; 26,55 ; 22,72 ;
- CH₃-12' :: 14,03

### EXEMPLE 6

Préparation du composé (I-a): X=CO ; R=C₁₁H₂₃ et Z₁ = Z₂ = H.

A une solution de 2,3 g (15 mmoles) d'oxychlorure de phosphore fraîchement distillé dans 40 ml de tétrahydrofuranne anhydre (système sodium/benzophénone), maintenue à 0°C sous agitation pendant 30 minutes, on ajoute une solution contenant 4,75 g (15 mmoles) de pyridine dans 60 ml de tétrahydrofuranne et, goutte à goutte, 3,29 g (10 mmoles) de 5-0-dodécanoyl-dianhydro-1,4:3,6-D-glucitol (composé III-a selon l'exemple 1) solubilisés dans 60 ml de tétrahydrofuranne. Après 1 heure à 0°C et 18 heures à 20°C, il se forme un précipité de chlorure de pyridinium qui est éliminé par filtration sur verre fritté (porosité 3).

Le filtrat obtenu, auquel on ajoute 80 ml d'un mélange eau/glace pilée, est agité (800RPM; 1 heure). Après l'ajout de 100ml d'un mélange chloroforme/méthanol 2/3 (v/v) suivi d'une phase de décantation, la phase organique blanchâtre est récupérée, traitée par une solution de potasse à 50 % en poids jusqu'à ce que le pH soit égal à 9,5. L'émulsion brune ainsi formée est concentrée dans un évaporateur rotatif en présence d'éthanol puis de toluène.

Le résidu d'évaporation, solubilisé dans l'eau, est chromatographié sur une colonne remplie de résine cationique forte (AMBERLITE IR 120 H⁺). Après concentration sous vide (40°C ; 13,3 Pa) de la solution issue de la colonne, on récupère 2 g de 5-0-dodécanoyl-dianhydro-1,4:3,6-D-glucitol-2-phosphate (I-a).

### EXEMPLE 7

Préparation du composé (I-b): X=CH₂, R=C₁₁H₂₃ et Z₁=Z₂=H.

On opère dans les conditions de l'exemple 5 en présence de 5-0-dodécyl-dianhydro-1,4:3,6-D-glucitol (composé III-b selon l'exemple 4) l'hydrolyse étant effectuée à l'aide de 10 ml d'eau. On récupère 0,37 g (rendement : 32 %) de 5-O-dodécyl-dianhydro-1,4:3,6-D-glucitol-2-phosphate (I-b).

Les caractéristiques du composé (I-b) sont présentées ci-après.

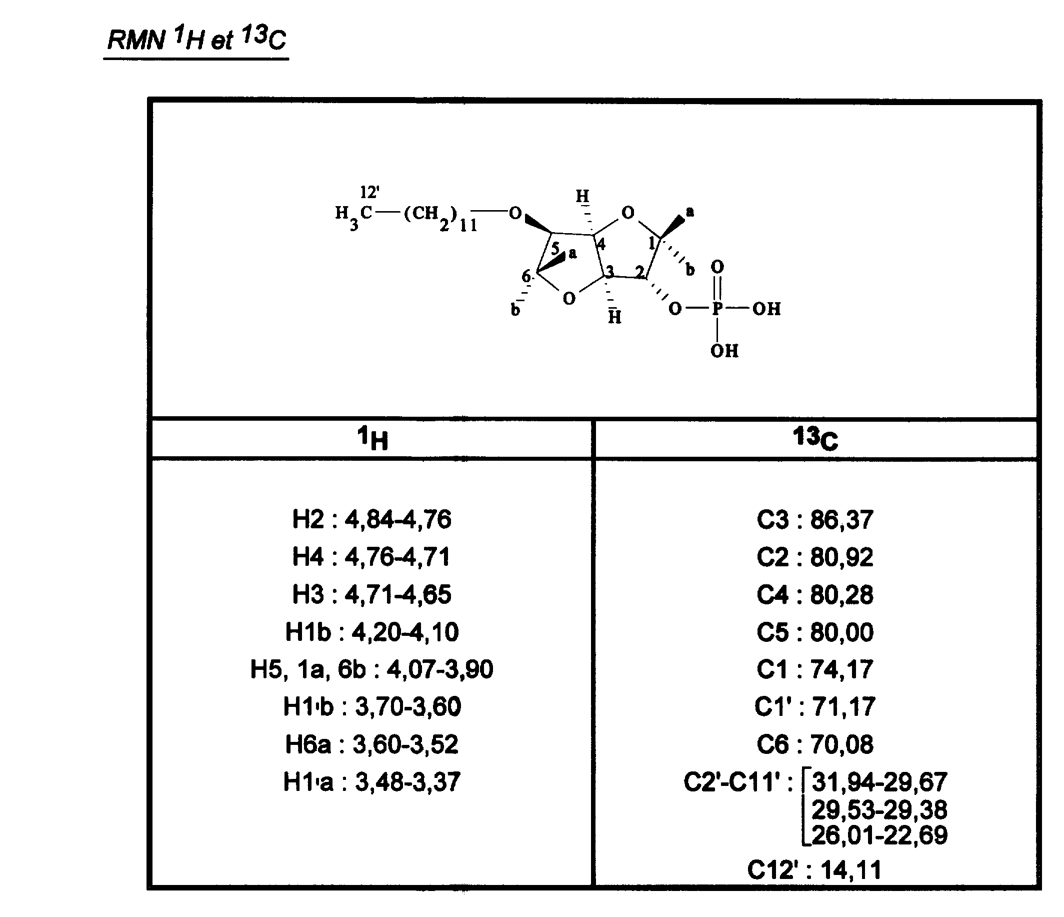

### EXEMPLE 8

Préparation du composé (II-b) : X=CH₂, R=C₁₁H₂₃ et Z₁=Z₂=H.

On opère dans les conditions de l'exemple 6 en présence de 2-O-dodécyl-dianhydro-1,4:3,6-D-glucitol (composé IV-b selon l'exemple 4).

On récupère 1,52 g (rendement : 40 %) de 2-O-dodécyl-dianhydro-1,4:3,6-D-glucitol-5-phosphate (Il-b).

Les caractéristiques du composé (Il-b) sont présentées ci-après.

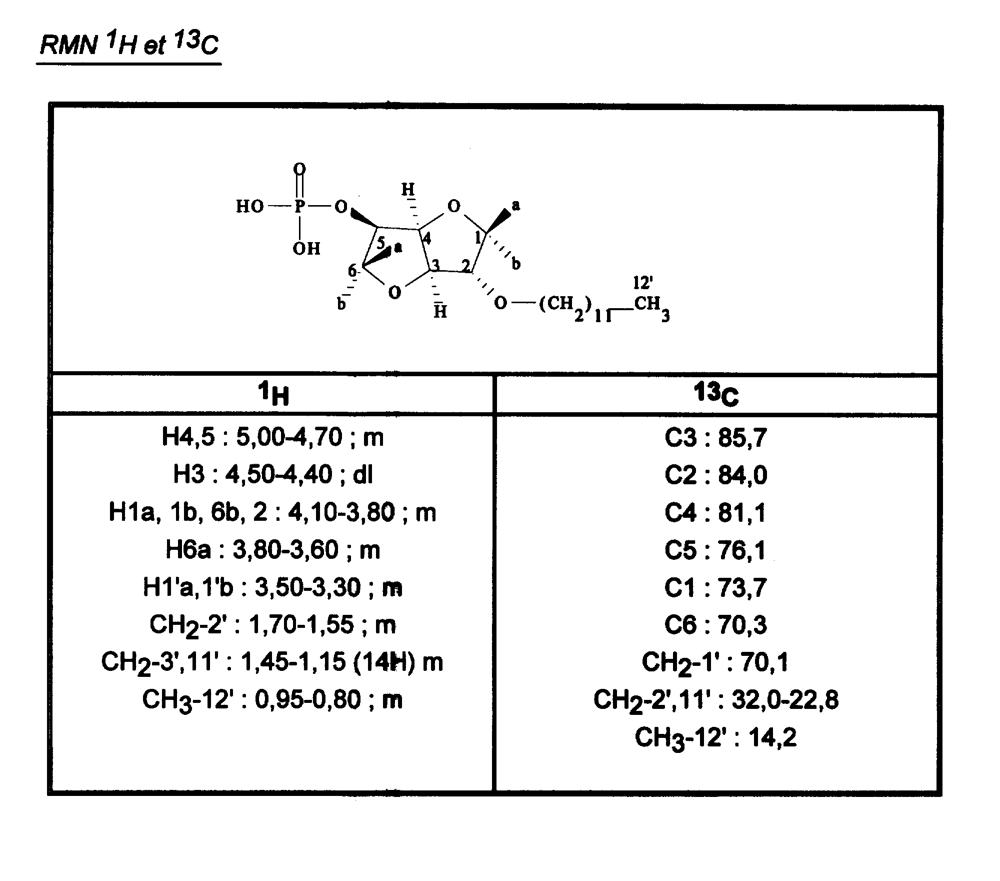

## Revendications

1. Composés de formule : 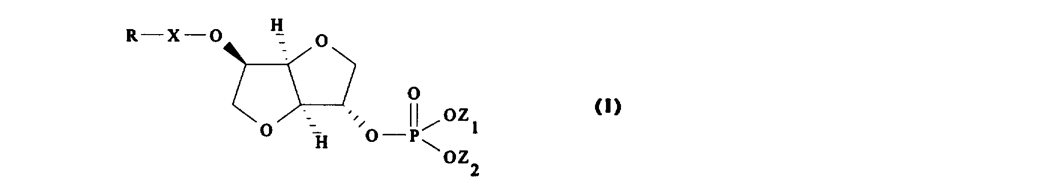 ou 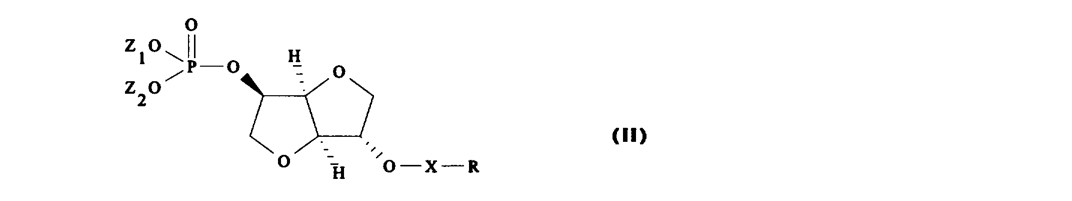 dans laquelle :
R représente un radical alkyle, linéaire ou ramifié, saturé ou insaturé, renfermant de 5 à 21 atomes de carbone,
X représente CO ou CH₂,
Z₁ et Z₂, identiques ou différents, représentent un atome d'hydrogène, un métal alcalin ou alcalino-terreux ou un ammonium quaternaire de formule : 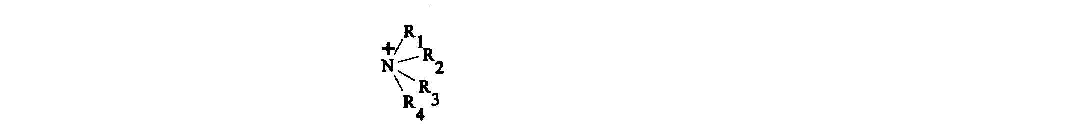 dans laquelle R₁, R₂, R₃ et R₄, identiques ou différents, représentent un atome d'hydrogène, un radical alkyle ou hydroxyalkyle renfermant de 1 à 6 atomes de carbone ou un résidu d'amino-acide basique.

2. Composés selon la revendication 1 dans lequel R renferme 6 à 17 atomes de carbones.

3. Procédé de préparation des composés selon la revendication 1 ou 2, caractérisé en ce qu'il consiste à :
**a)** faire réagir du dianhydro-1,4:3,6-D-glucitol avec un composé de formule RCOOR' ou RCH₂Y en présence d'un catalyseur pour former les composés de formule : 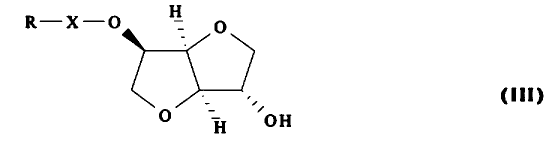 et 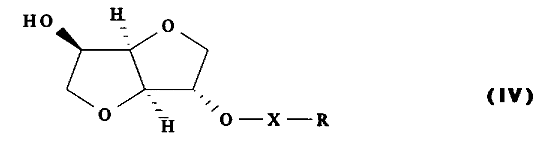 dans lesquelles :
Y représente un halogène, un radical alkyle ou arylsulfonique,
R' représente un atome d'hydrogène ou un radical alkyle ayant de 1 à 6 atomes de carbone
R représente un radical alkyle linéaire ou ramifié, saturé ou insaturé, renfermant de 5 à 21 atomes de carbone
X représente CO ou CH₂.
**b)** faire réagir le produit de l'étape a) avec de l'oxychlorure de phosphore, une base et un solvant anhydre, pour former les composés de formule (I) et/ou (Il),
et, éventuellement les séparer.

4. Procédé selon la revendication 3, caractérisé en ce que le rapport du nombre d'équivalents en mole de dianhydro-1,4:3,6-D-glucitol au nombre d'équivalents en mole du composé de formule RCOOR' est compris entre 0,1 et 20.

5. Procédé selon la revendication 3, caractérisé en ce que le rapport du nombre d'équivalents en mole de dianhydro-1,4:3,6-D-glucitol au nombre d'équivalents en mole du composé de formule RCH₂Y est compris entre 1 et 20.

6. Procédé selon l'une des revendications 3 à 5 caractérisé en ce que la base est choisie parmi la pyridine, la N-N-diméthyl ou diéthylamino-4 pyridine et les amines tertiaires.

7. Procédé selon l'une des revendications 3 à 6 caractérisé en ce que le nombre d'équivalents en mole d'oxychlorure de phosphore au nombre d'équivalents en mole du produit de l'étape a) est compris entre 1 et 5.

8. Utilisation des composés selon la revendication 1 ou 2 pour préparer des compositions destinées à l'hygiène.

9. Utilisation selon la revendication 8, caractérisée en ce que les compositions sont choisies parmi les shampooings et les compositions capillaires ou cosmétiques.

10. Utilisation selon la revendication 9, caractérisée en ce que les compositions cosmétiques appartiennent au groupe constitué par les pommades, les crèmes, les laits de beauté, les bains moussants, les gels de douche et les savons de type Syndet.

11. Utilisation des composés selon la revendication 1 ou 2 pour le traitement anti-statique des textiles.

12. Utilisation des composés selon la revendication 1 ou 2 pour le traitement des métaux.

13. Utilisation des composés selon la revendication 1 ou 2 dans les réactions de polymérisation en suspension.
